# EUROPEAN PATENT SPECIFICATION

(11) **EP 0 929 527 B1**
(45) Date of publication and mention of the grant of the patent: **27.10.2004**
(21) Application number: 97936381.9
(22) Date of filing: 04.08.1997
(51) Int. Cl.: C07D 217/06, C07D 221/20

(54) **PICTET-SPENGLER REACTION FOR THE SYNTHESIS OF TETRAHYDROISOQUINOLINES AND RELATED HETEROCYCLIC COMPOUNDS**
PICTET-SPENGLER REAKTION FÜR DIE HERSTELLUNG VON TETRAHYDROISOCHINOLINEN UND ANVERWANDTE HETEROCYCLISCHEN VERBINDUNGEN
REACTION DE PICTET-SPENGLER POUR LA SYNTHESE DES TETRAHYDRO-ISOQUINOLINES ET COMPOSES HETEROCYCLIQUES ASSOCIES

(30) Priority: 27.09.1996 US 722588
(43) Date of publication of application: 21.07.1999
(73) Proprietor: Aventis Pharmaceuticals Inc., Bridgewater, NJ 08807-0800 (US)
(72) Inventor: WATSON, Timothy, James-Norman, Centerville, OH 45458 (US)
(74) Representative: Minoja, Fabrizio, Dr.
(86) International application number: PCT/US1997/013672
(87) International publication number: WO 1998/013351

(56) References cited:
- KAZUO ITO ET AL: "Syntheses of 1,2,3,4-tetrahydroisoquinolines from N-sulfonyl-phenethylamines and aldehydes" CHEMICAL AND PHARMACEUTICAL BULLETIN., vol. 25, no. 7, July 1977, TOKYO JP, pages 1732-1739, XP002045380 cited in the application

## Description

### Background of the Invention:

The present application relates to a novel synthesis for certain tetrahydroisoquinolines which are useful intermediates in the preparation of certain cyclic nitrones which have multiple pharmaceutical utility, including for example, the prevention of oxidative tissue damage from oxygen based free radicals and the inhibition of interleuken-1. The utility of these cyclic nitrones and their advantages are better described in U.S. patent 5,292,746.

The Pictet-Spengler reaction is a condensation of a β-arylethylamine with a carbonyl compound to yield a tetrahydroisoquinoline, and is a specific example of the more general Mannich reaction. It has been generally accepted that the reactivity of the aromatic nucleus of the arylethylamine as well as the carbonyl reactant are significant to the success of the reaction. Whaley, W.M. & Govindachari, T.R., *Organic Reactions 6*: 151-190 (1951), the disclosure of which is herein incorporated by reference. Formaldehyde is routinely employed, as it is cheap, reactive and effective. More importantly, Whaley and Govindachari have noted that the activation of the aromatic ring, by some form of electrophilic substitution para to point of ring closure was necessary before the reaction will proceed.

The production of tetrahydroisoquinolines by way of Pictet-Spengler condensation of aryl N-sulfonylethylamines was described by K Ito and H. Tanaka in Chem. Pharm. Bull. 25(7), 1732-1739 (1977), the disclosure of which is herein incorporated by reference. The process conditions described the reaction in chloroform between N-sulfonatedphenethylamines with aqueous formaldehyde in the presence of BF₃-etherate. Because the formaldehyde was in an aqueous solution, and water is destructive of the BF₃-etherate, the BF₃-etherate must be used in substantial molar excess. This procedure is viable on a small laboratory scale, but is too inefficient and expensive to be applicable to a commercial scale synthesis.

### SUMMARY OF THE INVENTION

Applicants have created a process for the commercial scale production of tetrahydroisoquinolines and related heterocycles by reaction, in mildly acidic conditions, of aryl N-sulfonylethylamines in the presence of a Lewis acid, and a compound capable of *in situ* generation of formaldehyde. Applicants invention is an improvement upon the Ito and Tanaka process, in that water is not present as an initial reactant (the formaldehyde used was a 37% aqueous solution). Applicant's process is also characterized by formaldehyde being generated by the reaction of the Lewis acid (boron trifluoroetherate) upon the CH₂O generating agent, instead of being present as an initial reactant. The *in situ* generation of formaldehyde is advantageous because presently formaldehyde reagent is only available as a 37% aqueous solution, requiring a substantial molar excess of the Lewis acid to compensate for what which is deactivated by water.

The invention describes a process for creating a compound of the formula: 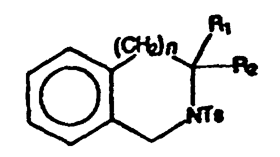 wherein R₁ and R₂ are each independently C₁₋₃ alkyl or R₁ and R₂ together form C₂₋₇ alkylene, n is an integer from 0-2, and Ts is *para*-toluenesulfonyl by reacting a compound of the formula: 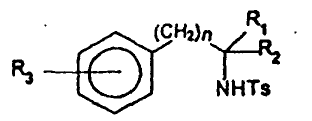 with a suitable Lewis acid in a formaldehyde generating solvent.

### DETAILED DESCRIPTION OF THE INVENTION

As used herein, the term "Lewis acid" means a strongly electrophilic compound capable of combining with another molecule by forming at least one covalent bond with two electrons from the second molecule. For example, borontrifluoro etherate (BF₃•OEt₂), aluminum chloride (AlCl₃), zinc chloride (ZnCl₂), magnesium bromide (MgCl₂), ferric chloride (FeCl₃). The preferred Lewis acid is borontrifluoro etherate.

As used herein, the term "formaldehyde generating solvent" means dimethoxy methane, diethoxymethane and, bis(methylthio)methane (CH₂(SCH₃)₂). The preferred formaldehyde generating agent is dimethoxy methane.

As used herein, the term "C₁₋₃ alkyl" means methyl, ethyl, *n*-propyl, isopropyl.

As used herein, the term "C₂₋₇ alkylene" means a straight chain alkyl bridge of two valences, such that the same atom does not have both valences. For example, ethylene, *n*-propylene, *n*-butylene, *n*-pentylene, *n*-hexylene, *n*-heptylene.

As used herein, the term 'halogen" means fluoro, chloro, bromo, iodo.

The present invention is a useful part of a multi-step synthesis for the creation of certain cyclic nitrones useful in the prevention of oxidative tissue damage and which are described in U.S. patent 5,292,746. More particularly, this synthesis may be carried out as follows:

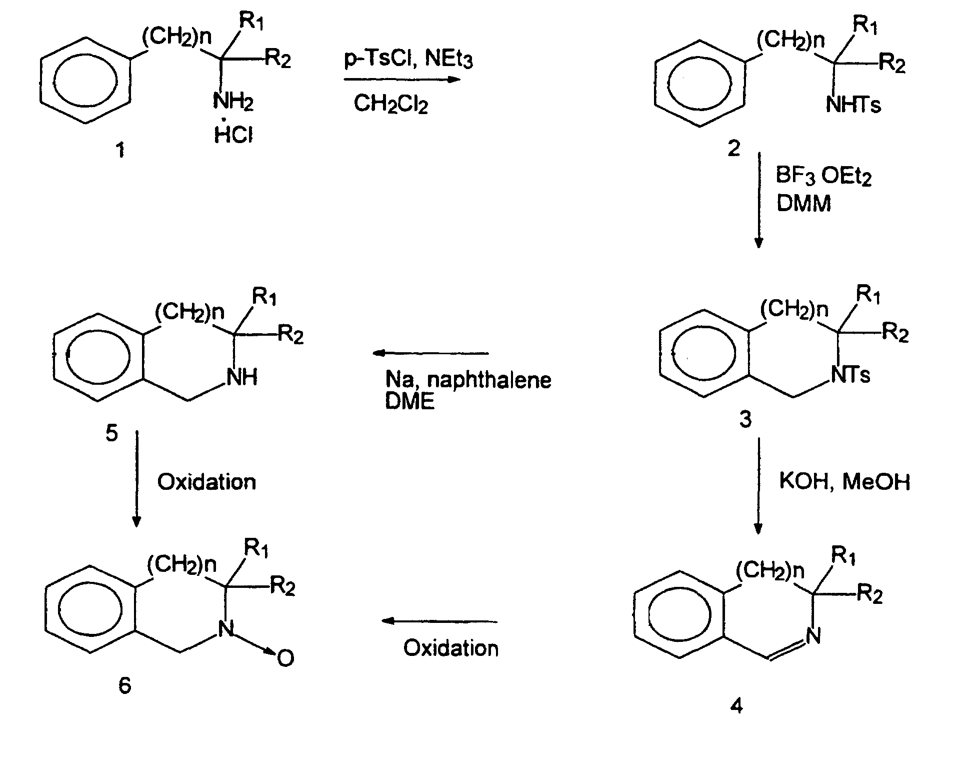

In the above scheme, the tosylated amine [2] may be preparated, as indicated by Ito and Tanaka, in the presence of triethylamine (NEt₃). The tosylated amine may then be converted into the tetrahydroisoquinoline or related heterocyclic [3] as described in the present invention. This compound, as is known, can then be subjected to alkaline conditions in KOH/MeOH to afford the dihydro analog [4], or treated with sodium naphthalenide in DME to give the unprotected cyclic amine [5]. Compound [4] or [5] may then be oxidized into the nitrone [6], for example, by application of sodium tungstate (NaWO₄, as is described in "Synthesis and Radical Scavenging Activity of 3,3-Dialkyl-3,4-Dyhydro-Isoquinoline-2-Oxides", Biorganic & Medicinal Chemistry Letters, (in press), Berotas, R.C. et al.

The following examples are given to illustrate in further details the practice of the invention. The following compounds can be prepared 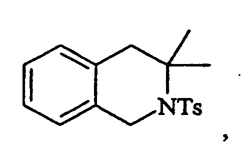 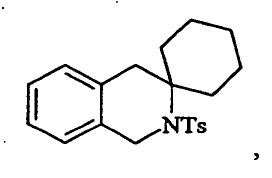 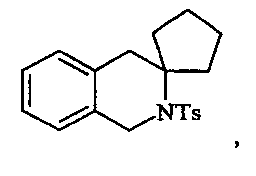 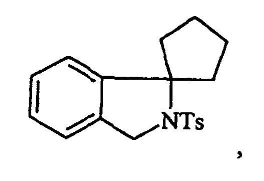 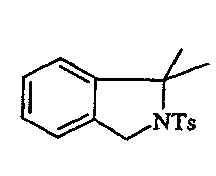 and

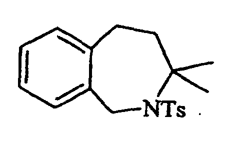

### Example 1

To a nitrogen-blanketed solution of 1,1-dimethyl-2-phenyl-ethylamine (3.56 g, 0.019 mol), methylene chloride (CH₂Cl₂, 20 mL) and triethylamine (Et₃N, 8.01 mL, 0.058 mol) was added para-toluenesulfonyl chloride (TsCl, 4.39 g, 0.023 mol). The mixture was stirred at room temperature for 12 hours while monitoring by gas chromatography. The reaction mixture was partitioned between methylene chloride (100 mL) and water (100 mL) and the organic layer was separated and dried over sodium sulfate. The drying agent was filtered off and the filtrate was concentrated to give 5.93 g of N-toluenesulfonyl-1,1-dimethyl-2-phenyl-ethylamine (yield = 99%).
IR (Kbr, cm-1) 3443, 3283, 1311, 1097;
¹H-NMR (300 MHz, CDCl₃) δ 7.72 (m, 2H), 7.21-7.35 (m, 7H), 4.50 (bs, 1H), 2.83 (s, 2H), 2.40 (s, 3H), 1.18 (s, 6H);
¹³C-NMR (75 MHz, CDCl₃) ppm 142.8, 140.6, 136.6, 130.8, 129.4, 128.2, 126.9, 126.7, 56.9, 49.0, 27.4, 21.5;
MS *m*/*z* (M⁺) calc'd 303.4, observed 304.
Analysis calc'd for C₁₇H₂₁NO₂S: C, 67.30; H, 6.98; N, 4.62. Found: C, 67.23; H, 6.90; N, 4.55.

### Example 2

To a nitrogen-blanketed mixture of N-toluenesulfonyl-1,1-dimethyl-2-phenylethylamine (8.30 g, 0.027 mol.) in dimethoxymethane (50 mL) was added boron trifluororo etherate (BF₃•OEt₂, 9.9 mL, 0.081 mol.). The mixture was stirred at room temperature for 12 hours while monitoring by gas chromatography. The reaction mixture was partitioned between ethyl acetate (100 mL) and water (100 mL), separated, and the organic layer is washed with saturated sodium bicarbonate (2 x 100 mL) and dried over sodium sulfate (Na₂SO₄). The drying agent was filtered off and the filtrate was concentrated at 40°/50 torr to give 8.55 g of N-toluenesulfonyl-2,2-dimethyl-1,2,3,4-tetrahydroisoquinoline (yield = 99%).
IR (KBr, cm⁻¹) 3441, 2984, 1338, 1159;
¹H-NMR (300 Mhz, CDCl₃) δ 7.65 (m, 2H), 7.05-7.25 (m, 7H), 4.59 (s, 2H), 2.39 (s, 3H), 1.40 (s, 6H);
¹³C-NMR (75 Mhz, CDCl₃) ppm 142.7, 139.7, 134.5, 133.6, 129.4, 128.1, 127.2, 126.9, 126.4, 125.4, 58.1, 46.9, 44.9, 27.7, 21.4;
MS *m*/*z* (M⁺) calc'd 315.4, observed 315.
Anal. calc'd for C₁₈H₂₁NO₂S: C, 68.54; H, 6.71; N, 4.44. Found: C, 68.14; H, 6.70; N, 4.37.

### Example 3

To a nitrogen-blanketed mixture of potassium hydroxide (KOH, 30 g) and methanol (CH₃OH, 60 mL) was added of N-toluenesulfonyl-3,3-dimethyl-1,2,3,4-tetrahydroisoquinoline (4.0 g, 0.013 mol.). The reaction mixture was heated at reflux for 17 hours and the reaction was followed by gas chromatagraphy. The reaction mixture was cooled to ambient temperature, quenched with water (100 mL) and 10% HCl was slowly added until pH=7 was obtained. The aqueous mixture was extracted with methylene chloride (3 x 100 mL) and the organic layers were combined and stirred with charcoal and sodium sulfate (Na₂SO₄). The solution was filtered through celite and the filtrate was concentrated (25°/150 torr) to give 2,2-dimethyl-3,4-dihydroisoquinoline (1.79 g, yield = 90%).
IR (neat, cm⁻¹) 3389, 2966, 1628;
¹H-NMR (300 Mhz, CDCl₃) δ 8.23 (s, 1 H), 7.40-7.15 (m, 4H), 2.72 (s, 2H), 1.25 (s, 6H);
¹³C-NMR (75 Mhz, CDCl₃) ppm 157.4, 135.6, 131.0, 128.0, 127.5, 127.0, 126.9, 54.7, 37.9, 28.0;
MS *m*/*z* (M⁺) calc'd 159.23, observed 159.

### Example 4

To a stirred solution of naphthalene (5.8 g, 0.045 mol) in diethoxymethane (50 mL) was added sodium metal (1.09 g, 0.039 mol.). The mixture was allowed to stir for four (4) hours until a dark green color persisted. To this was added of N-toluenesulfonyl-3,3-dimethyl-1,2,3,4-tetrahydroisoquinoline (5.0 g, 0.016 mol.) in 20 mL of dimethoxymethane. The reaction was monitored by gas chromatography. When the reaction was complete (≈2 hours), the mixture was quenched with saturated sodium chloride (70 mL). The mixture was partitioned between ethyl acetate (250 mL) and 10% HCL (250 mL) and the organic layer was discarded. 10% Sodium hydroxide was added to the aqueous layer until a pH=7 was obtained. The aqueous layer was further extracted over methylene chloride (2 x 100 mL), dried over magnesium sulfate, filtered and concentrated (25°C/150 torr) to produce 2.2 g (86%) of 3,3-dimethyl-1,2,3,4-tetrahydroisoquinoline.
IR (neat, cm⁻¹) 3043, 2897, 744;
¹H-NMR (300 Mhz, CDCl₃) δ 7.14-7.00 (m, 4H), 4.02 (s, 2H), 2.61 (s, 2H), 1.58 (bs, 1H), 1.19 (s, 6H);
¹³C-NMR (75 Mhz, CDCl₃) 134.5, 134.4, 129.5, 125.9, 125.6, 125.5, 48.6, 44.3, 41.5, 27.7; MS *m*/*z* (M⁺) calc'd 161.24, observed 161.

## Claims

1. A process for preparing a compound of the formula: 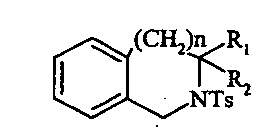 wherein R₁ and R₂ are each independently C₁₋₃ alkyl or R₁ and R₂ together form C₂₋₇ alkylene, n is an integer from 0 to 2 and Ts is *para*-toluenesulfonyl, by reacting a compound of the formula: 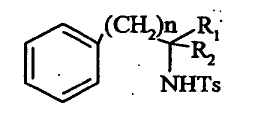 with a Lewis acid in a formaldehyde generating solvent selected from dimethoxymethane, diethoxymethane, and bis(methylthio)methane, under mildly acid conditions.

2. The process of claim 1 wherein the Lewis acid is selected from the group consisting of boron trifluoroetherate (BF₃· OEt₂), aluminum chloride, zinc chloride, magnesium bromide, and ferric chloride (FeCl₃).

3. The process of claim 2 wherein the Lewis acid is boron trifluoroetherate.

4. The process of claim 3 wherein the formaldehyde generating solvent is dimethoxymethane.

5. The process of any one of claims 1 to 4 for preparing the compound wherein R₁ and R₂ are each C₁₋₃ alkyl.

6. The process of any one of claims 1 to 5 for preparing the compound wherein n is 0 or 1.

7. The process of any one of claims 1 to 5 for preparing the compound wherein n is 1 and R₁ and R₂ are each methyl or R₁ and R₂ taken together represent -(CH₂)₅- or-(CH₂)₄-.

8. The process of claim 1 wherein the obtained compound is selected from those having the following formulae: 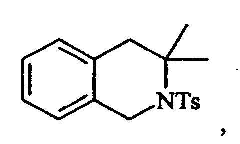 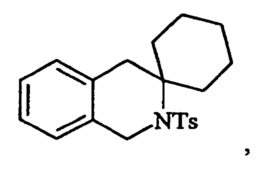 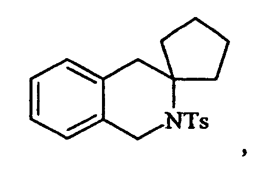 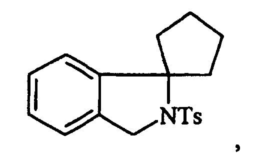 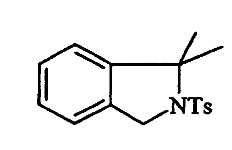 and

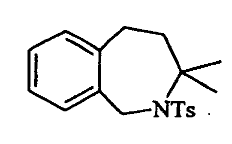

## Patentansprüche

1. Verfahren zur Herstellung einer Verbindung der Formel: 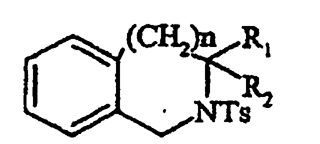 worin R₁ und R₂ jeweils unabhängig C₁₋₃-Alkyl sind oder R₁ und R₂ gemeinsam C₂₋₇-Alkylen bilden, n eine ganze Zahl von 0 bis 2 ist, und Ts para-Toluolsulfonyl ist, durch Umsetzung einer Verbindung der Formel 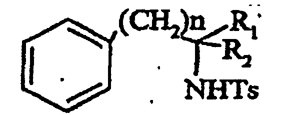 mit einer Lewis-Säure in einem Formaldehyd-bildenden Lösungsmittel, ausgewählt unter Dimethoxymethan, Diethoxymethan, und Bis-(methylthio)-methan, unter schwach sauren Bedingungen.

2. Verfahren gemäß Anspruch 1, worin die Lewis-Säure aus der Gruppe, bestehend aus Bortrifluorid-Etherat (BF₃^{·}OEt₂), Aluminiumchlorid, Zinkchlorid, Magnesiumbromid und Ferrichlorid (FeCl₃) ausgewählt wird.

3. Verfahren gemäß Anspruch 2, worin die Lewis-Säure Bortrifluorid-Etherat ist.

4. Verfahren gemäß Anspruch 3, worin das Formaldehyd-bildende Lösungsmittel Dimethoxymethan ist.

5. Verfahren gemäß einem der Ansprüche 1 bis 4 zur Herstellung der Verbindung, worin R₁ und R₂ jeweils C₁₋₃-Alkyl sind.

6. Verfahren gemäß einem der Ansprüche 1 bis 5 zur Herstellung der Verbindung, worin n für 0 oder 1 steht.

7. Verfahren gemäß einem der Ansprüche 1 bis 5 zur Herstellung der Verbindung, worin n für 1 steht und R₁ und R₂ jeweils Methyl sind oder R₁ und R₂ gemeinsam -(CH₂)₅- oder -(CH₂)₄- bedeuten.

8. Verfahren gemäß Anspruch 1, worin die erhaltene Verbindung unter denjenigen der Formeln 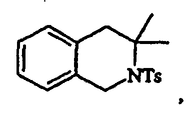 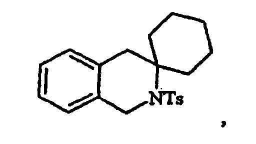 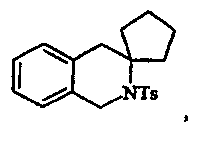 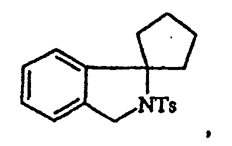 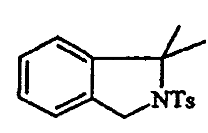 und 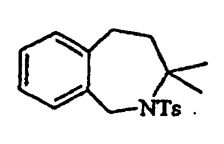 ausgewählt wird.

## Revendications

1. Procédé pour préparer un composé de formule : 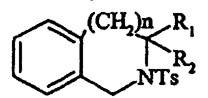 dans laquelle R₁ et R₂ sont chacun indépendamment un alkyle en C₁₋₃ ou R₁ et R₂ forment ensemble un alkylène en C₂₋₇, n est un nombre entier de 0 à 2 et Ts est un para-toluènesulfonyle, en faisant réagir un composé de formule : 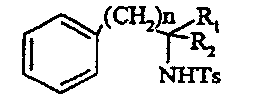 avec un acide de Lewis dans un solvant générant du formaldéhyde, choisi parmi le diméthoxyméthane, le diéthoxyméthane et le bis(méthylthio)méthane, dans des conditions légèrement acides.

2. Procédé selon la revendication 1, **caractérisé en ce que** l'acide de Lewis est choisi parmi le groupe comprenant le trifluoroéthane de bore (BF₃ . OEt₂), le chlorure d'aluminium, le chlorure de zinc, le bromure de magnésium et le chlorure ferrique (FeCl₃).

3. Procédé selon la revendication 2, **caractérisé en ce que** l'acide de Lewis est le trifluoroéthane de bore.

4. Procédé selon la revendication 3, **caractérisé en ce que** le solvant générant du formaldéhyde est le diméthoxyméthane.

5. Procédé selon l'une quelconque des revendications 1 à 4 pour préparer le composé, **caractérisé en ce que** R₁ et R₂ sont chacun un alkyle en C₁₋₃.

6. Procédé selon l'une quelconque des revendications 1 à 5 pour préparer le composé, **caractérisé en ce que** n est 0 ou 1.

7. Procédé selon l'une quelconque des revendications 1 à 5 pour préparer le composé **caractérisé en ce que** n est 1 et R₁ et R₂ sont chacun un méthyle ou R₁ et R₂ pris ensemble représentent -(CH₂)₅- ou - (CH₂)₄-.

8. Procédé selon la revendication 1 **caractérisé en ce que** le composé obtenu est choisi parmi ceux ayant les formules suivantes: 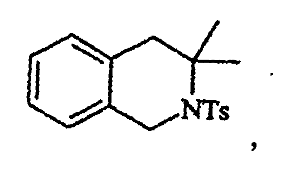 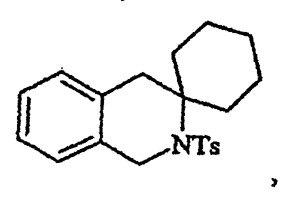 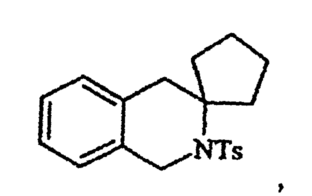 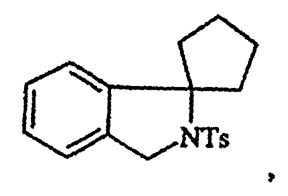 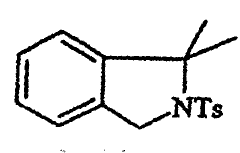 and
